# EUROPEAN PATENT SPECIFICATION

(11) **EP 2 310 858 B1**
(45) Date of publication and mention of the grant of the patent: **25.02.2015**
(21) Application number: 09776962.4
(22) Date of filing: 03.07.2009
(51) Int. Cl.: G01N 33/564, G01N 33/68

(54) **THERAPEUTIC AND DIAGNOSTIC METHODS FOR ALZHEIMER'S DISEASE**
THERAPEUTISCHE UND DIAGNOSTISCHE VERFAHREN FÜR MORBUS ALZHEIMER
PROCÉDÉS THÉRAPEUTIQUES ET DIAGNOSTIQUES POUR LA MALADIE D'ALZHEIMER

(30) Priority: 07.07.2008 US 78677 P
(43) Date of publication of application: 20.04.2011
(73) Proprietor: Athera Biotechnologies AB, 113 43 Stockholm (SE)
(72) Inventor: FROSTEGÅRD, Johan, S-112 42 Stockholm (SE); DE FAIRE, Ulf, S-187 33 Täby (SE); PEDERSEN, Nancy, S-171 70 Solna (SE)
(74) Representative: Potter Clarkson LLP
(86) International application number: PCT/EP2009/004849
(87) International publication number: WO 2010/003602

(56) References cited:
- WO-A-2005/100405
- US-A1- 2004 197 831
- WEKSLER MARC E ET AL: "Do age-associated changes in 'physiologic' autoantibodies contribute to infection, atherosclerosis, and Alzheimer's disease?" EXPERIMENTAL GERONTOLOGY 2002 AUG-SEP, vol. 37, no. 8-9, August 2002 (2002-08), pages 971-979, XP002552512 ISSN: 0531-5565
- SU J ET AL: "Antibodies of IgM subclass to phosphorylcholine and oxidized LDL are protective factors for atherosclerosis in patients with hypertension" ATHEROSCLEROSIS, ELSEVIER IRELAND LTD, IE, vol. 188, no. 1, 1 September 2006 (2006-09-01), pages 160-166, XP025242667 ISSN: 0021-9150 [retrieved on 2006-09-01]

## Description

### FIELD OF THE INVENTION

This invention relates to the fields of medicine and biology. In particular, it relates to the risk assessment and diagnosis of Alzheimer's disease.

### BACKGROUND

The listing or discussion of an apparently prior-published document in this specification should not necessarily be taken as an acknowledgement that the document is part of the state of the art or is common general knowledge.

Alzheimer's disease (AD) is a progressive neurodegenerative disease that afflicts approximately 1% of the population over the age of 65. Characteristic features of the disease include neurofibrillary tangles composed of abnormal tau protein paired helical filaments, neuronal loss, and alteration in multiple neurotransmitter systems. A significant pathological feature is an overabundance of diffuse and compact senile plaques in association with limbic areas of the brain. Although these plaques contain multiple proteins, their cores are composed primarily of β-amyloid, a 39-42 amino acid proteolytic fragment derived from amyloid precursor protein (APP). Deposition of β-amyloid in mammalian brain is a defining feature of Alzheimer's disease, and there is evidence that activation of inflammatory pathways is important in the pathogenesis of the disease.

Phosphorylcholine (PC) is a major component not only in inflammatory phospholipids like platelet activating factor-PAF (where it is essential for interaction with the PAF-receptor) and in oxLDL, but is also as an immunogenic components of many bacteria including *S*. *pneumoniae.* Furthermore, PC is expressed by apoptotic cells (Binder et al., 2002. Nature Medicine 8, 1218-26).

Weksler ME, Goodhardt M., 2002, Exp Gerontol., 37, 971-979 discusses evidence that autoantibodies may influence the risk of the elderly developing infectious, atherosclerotic, or Alzheimer's disease. The authors report that auto-anti-idiotypic antibodies suppress the antibody response to the nominal antigen and, thus, may contribute to the increased risk of infection and poor response to vaccines in the elderly, whereas in contrast, it is taught that low levels of autoantibodies to oxidised low-density lipoproteins may contribute to the increased risk of developing atherosclerosis and that low levels of autoantibodies to amyloid beta peptide may contribute to the increased risk of developing Alzheimer's disease.

US 2004/197831 (A1) describes a method for assessing risk of a neurodegenerative disease or disorder in a subject. The method comprises comparing a level of anti-amyloid peptide antibody in a biological sample from a subject to a normal level, wherein a lower level in the biological sample from the subject indicates the presence of the disease or disorder. In a specific embodiment, the disease or disorder is Alzheimer's Disease (AD); in a further specific embodiment, the amyloid peptide is beta-amyloid-42 (Abeta42). In addition, US 2004/197831 (A1) reports that certain neurodegenerative diseases or disorders are associated with a deficiency of anti-amyloid antibodies, and teaches a method of treating such a disease or disorder in a subject, by administering a therapeutically effective amount of a human anti-amyloid peptide antibody to a subject believed to suffer from the immune deficiency or disorder. For example, the disease or disorder can be Alzheimer's Disease (AD). In such an embodiment, the amyloid peptide can be beta-amyloid-42 (Abeta42).

The existence of antibodies against PC (anti-PC) in humans has been known for decades (Shaw et al., 2000. J Clin Invest 105, 1731-1740) and have been linked to pneumonia (Nordenstam et al., 1990. Scand J Infect Dis 22, 187-195) and also to oral pathogens and gingivitis (Schenkein et al., 1999. Infect Immun 67, 4814-4818. PC is an immunodominant determinant of pneumococcal teichoic acids. It has been known for some time that immunization with PC-conjugates can protect mice against lethal infection with bacteria like *S*. *pneumoniae* (Briles et al., 1982. J Exp Med 156, 1177-1185).

Antibodies to PC (anti-PC) are present in normal healthy individuals and could therefore be described as natural antibodies, which are conserved evolutionarily and similar antibodies are found in mice (Brown et al., 1984. J Immunol 132, 1323-1328).

In U.S. Patent 5,455,032, PC-conjugates have been used in vaccines for inducing immunoprotection against infections such as *Streptococcus pneumoniae.* In a study by Binder et al, 2003 (Nature Medicine 9, 736-43) on pneumococcus vaccine in mice, it was also shown that vaccination decreased atherosclerotic lesion formation. It was found that many autoantibodies to oxLDL derived from atherosclerotic mice share structural identity with antibodies which protect against common infectious pathogens, including *Streptococcus pneumoniae.*

In another study (Zanchetti et al., 1998. J Hypertens 16, 949-61), it was shown that anti-PC antibody (anti-PC) serum levels are elevated in humans with periodontal diseases. The conclusion is that PC is an important oral antigen associated with organisms in the periodontal flora and that the levels of PC antibodies are elevated as a consequence of periodontal disease. High serum levels of PC antibodies (anti-PC) have been shown to be a protective factor for atherosclerosis in patient with hypertension (Su et a/., 2006. Atherosclerosis 188, 160-6), decreased serum levels of PC antibodies (anti-PC) have been shown to be related to increased risk of cardiovascular disease (WO 2005/100405).

Passive immunization with antibodies directed to a PC-conjugate has been shown to reduced development of atherosclerosis in apoE -/-mice (Faria-Neto et a/., 2006. Atherosclerosis 189, 83-90). Recently, active immunization with a PC-conjugate has been shown to reduce development of atherosclerosis in apoE -/- mice (Caliguiri et al., 2007. J Am Coll Cardiol 50, 540-546).

### SUMMARY OF THE INVENTION

The present inventors have surprisingly found that low levels of antibodies reactive with a PC-conjugate (anti-PC) are related to an increased risk of developing, or progression of, Alzheimer's disease.

Thus, the invention provides a method of diagnosing the absence, presence and/or levels of IgM, IgG or IgA antibodies with reactivity to PC and/or a PC-conjugate related to increased or decreased risk of developing Alzheimer's diseases in an *ex vivo* sample taken from a human patient, using a PC-conjugate, and further to the use of this information to determine whether an individual is at risk of developing Alzheimer's disease, and/or is at risk of actually having already developed Alzheimer's disease.

Accordingly, a first aspect of the invention provides a method for assessing a human patient's risk of developing or progression of Alzheimer's disease, the method comprising assessing the patient's level of antibodies reactive with phosphorylcholine (PC) and/or a PC-conjugate in an *ex vivo* sample taken from the human patient, wherein the PC-conjugate comprises phosphorylcholine linked to a carrier, optionally via a spacer, and wherein the carrier is a protein, a carbohydrate, a lipid, a polymer, latex beads, or colloid metal. Low levels of the antibodies reactive with phosphorylcholine (PC) and/or a PC-conjugate are predictive of increased risk of developing or progression of Alzheimer's disease. The method may, for example, assess the level of IgM, IgG and/or IgA antibodies reactive with phosphorylcholine (PC) and/or a PC-conjugate. The method may, for example, assess the level of antibodies reactive with phosphorylcholine (PC) and/or a PC-conjugate by using a phosphorylcholine conjugate.

In a second aspect, the present invention provides a method of diagnosing the presence or absence of IgM, IgG or IgA antibodies reactive with phosphorylcholine (PC) and/or a PC-conjugate related to increased or decreased risk of developing Alzheimer's disease in an *ex vivo* sample taken from the human patient, using a phosphorylcholine conjugate, wherein the PC-conjugate comprises phosphorylcholine linked to a carrier, optionally via a spacer, and wherein the carrier is a protein, a carbohydrate, a lipid, a polymer, latex beads, or colloid metal. Low levels of the IgM, IgG or IgA antibodies related to increased or decreased risk of developing Alzheimer's disease are predictive of increased risk of developing or progression of Alzheimer's disease.

In methods according to the first and/or second aspects of the present invention the PC-conjugate may or may not comprise phosphorylcholine linked to a carrier via a spacer. The PC-conjugate may or may not comprise phosphorylcholine linked to a carrier protein, such as KLH (keyhole limpet hemocyanin), human serum albumin (HSA) or bovine serum albumin (BSA).

In methods according to the first and/or second aspects of the present invention the assay may be an immunoassay.

In methods according to the first and/or second aspects of the present invention, the test sample may, for example, be taken from a human patient aged at least 40, 50, 60, 65, 70, 75, 80, 85 of more years.

Accordingly, in a third aspect of the present invention, there is provided a use of a phosphorylcholine conjugate in a method for assessing an individual's risk of developing or progression of Alzheimer's disease in which the individual's levels of antibodies, such as IgM, IgG or IgA antibodies, reactive with the phosphorylcholine conjugate are assessed, wherein the phosphorylcholine conjugate comprises phosphorylcholine linked to a carrier, optionally via a spacer, and wherein the carrier is a protein, a carbohydrate, a lipid, a polymer, latex beads, or colloid metal. The use of the third aspect may, therefore, be in the assessment of the levels of antibodies reactive with the phosphorylcholine conjugate in accordance with a method of the first and/or second aspects of the invention.

Thus, as disclosed herein, the present invention provides for the use of a at least one PC-conjugate, or an antibody preparation, for example a monoclonal antibody, with reactivity to PC and/or a PC-conjugate, in the manufacture of a medicament for immunization and prophylaxis, prevention and/or treatment of Alzheimer's disease. The medicament is intended to provide active (where the composition comprises at least one PC-conjugate) or passive (where the composition comprises the defined antibody) immunization having immunogenic or therapeutic properties against Alzheimer's disease.

In other words, the invention describes at least one PC-conjugate, or an antibody preparation (for example a monoclonal antibody) with reactivity to PC and/or a PC-conjugate, for use in the prophylaxis, prevention and/or treatment of Alzheimer's disease.

Also described herein is a method for immunization and treatment against Alzheimer's disease, the method comprising the step of administering to a subject a pharmaceutical composition comprising at least one PC-conjugate, or an antibody preparation (for example a monoclonal antibody) with reactivity to PC and/or a PC-conjugate. The pharmaceutical composition is intended to provide active or passive immunization having immunogenic or therapeutic properties against Alzheimer's disease. The pharmaceutical preparation may be intended for administration by injection.

By a PC-conjugate is meant a PC moiety linked to a carrier, optionally via a spacer, wherein the carrier is a protein, a carbohydrate, a lipid, a polymer, latex beads, or colloid metal. The structural element PC may, or may not, comprise a derivative of PC. The PC-conjugate may for example be a protein-PC conjugate, such as a human serum albumin (HSA)-PC conjugate , a keyhole limpet hemocyanin (KLH)-PC conjugate or a bovine serum albumin (BSA)-PC conjugate. Examples of PC-conjugates and generation of anti-PC antibodies are, *e.g.,* described in WO 2005/100405 and U.S. Patent 5,455,032.

The invention also describes the use of one or more of the PC-conjugates as defined in relation to the preceding aspects of the invention, in the manufacture of a pharmaceutical composition, optionally in combination with an adjuvant, for immunotherapy or therapy for the prevention, prophylaxis and/or treatment of Alzheimer's disease.

Also described herein is a method of prophylactic or therapeutic treatment of a subject suffering from Alzheimer's disease or facing the risk of developing Alzheimer's disease, whereby a therapeutically effective amount of at least one PC-conjugate or an antibody preparation, for example a monoclonal antibody, with reactivity to PC and/or a PC-conjugate is administered.

The invention also provides methods to determine the presence or absence of antibodies, for example IgM, IgG or IgA antibodies, with reactivity to a PC-conjugate which are related to an increased or decreased risk of developing Alzheimer's diseases, in an *ex vivo* sample taken from a human patient.

Accordingly, the invention also provides a method of diagnosing the presence or absence of antibodies, for example IgM, IgG or IgA antibodies, related to increased or decreased risk of developing Alzheimer's diseases, in an *ex vivo* sample taken from a human patient, using a PC-conjugate.

Thus, the invention also provides the use of a PC-conjugate in a method for assessing an human individual's risk of developing or progression of Alzheimer's disease in which the individual's levels of antibodies, for example IgM, IgG or IgA antibodies, with reactivity to PC and/or to the PC-conjugate are assessed in an *ex vivo* sample taken from the individual.

The individual's levels of antibodies, *e.g.*, IgM, IgG or IgA antibodies, with reactivity to PC and/or the PC-conjugate may be assessed using an immunoassay. Examples of suitable immunoassays are described below and will in any case be apparent to those skilled in the art.

Throughout this application, the term "about" is used to indicate that a value includes the standard deviation of error for the device or method being employed to determine the value. Alternatively, it may be used to signify a value that is ± 20, 10, 5, 4, 3, 2, 1 or less than 1% of the stated value.

As used herein the specification, "a" or "an" may mean one or more. As used herein in the claim(s), when used in conjunction with the word "comprising", the words "a" or "an" may mean one or more than one. As used herein "another" may mean at least a second or more.

The use of the term "or" in the claims is used to mean "and/or" unless explicitly indicated to refer to alternatives only or the alternative are mutually exclusive, although the disclosure supports a definition that refers to only alternatives and "and/or."

Other objects, features and advantages of the present invention will become apparent from the following detailed description. It should be understood, however, that the detailed description and the specific examples, while indicating preferred embodiments of the invention, are given by way of illustration only.

### DETAILED DESCRIPTION OF THE INVENTION

### I. DIAGNOSIS AND RISK ASSESSMENT

The present invention is based on the surprising finding that low levels of antibodies reactive with a PC-conjugate (anti-PC) are related to an increased risk of developing Alzheimer's disease, and to an increased risk of actually having already developed Alzheimer's disease.

Accordingly, methods that allow for the determination of absence, presence and/or levels of antibodies with reactivity to PC and/or a PC-conjugate can be used as an early-warning mechanism (i.e. a predictive method) for the likelihood of developing Alzheimer's disease and/or as a marker of the presence of the disease.

As discussed above, an aspect of the invention is to provide a method of diagnosing the absence, presence and/or levels of antibodies, for example IgA, IgM or IgG antibodies, with reactivity towards PC and/or a PC-conjugate (that is, anti-PC antibodies) which factor is related to an increased or decreased risk of developing Alzheimer's diseases, using a PC-conjugate and to the use of this information to determine whether an individual is at risk of developing Alzheimer's disease, and/or is at risk of actually having already developed Alzheimer's disease. A preferred method is an immunoassay. The method may be used in assessing an individual's risk of developing or progression of Alzheimer's disease and/or may be used to monitor the efficacy of the treatment methods of the invention by active or passive immunisation, insofar as they are directed at increasing the anti-PC titre in an individual in order to effect prophylaxis, prevention and/or treatment of Alzheimer's disease.

The method of diagnosis will be performed on an *ex vivo* sample taken from the human test subject. The sample may, for example, be an *ex vivo* serum sample or an *ex vivo* plasma sample. The human subject from which the test sample is taken may, for example, be aged at least 40, 50, 60, 65, 70, 75, 80, 85 of more years.

The individual's levels of antibodies, e.g., IgM, IgG or IgA antibodies, with reactivity to PC and/or the PC-conjugate may, for example, be assessed by exposing a PC-conjugate to a sample from test subject and detecting antibodies which have bound to the PC-conjugate. Thus, antibody levels may be determined using an immunoassay. Examples of suitable immunoassays are described below and will in any case be apparent to those skilled in the art.

Preferably, when used in methods of diagnosis according to the present invention, PC is linked to a carrier via a spacer. In this embodiment, typically the carrier is a protein, preferably KLH (keyhole limpet hemocyanin), transferrin, human serum albumin (HSA) or bovine serum albumin (BSA). Alternatively, the carrier may be latex beads.

Levels of antibodies may be characterised by assaying for all antibodies with reactivity to a PC and/or the PC-conjugate, or for only antibodies of a particular isotype, such as IgM, IgG or IgA, or for a combination of two or more antibody isotypes. In one embodiment, the level of IgM is determined.

Immunoassays can be competitive or noncompetitive. In a typical competitive immunoassay, the antibody in the sample competes with labeled antibody to bind with the PC and/or the PC-conjugate. The amount of labeled antibody bound to the PC and/or the PC-conjugate is then measured. There is an inverse relationship between concentration of antibody in the sample and the quantity of labeled antibody detected. In noncompetitive immunoassays, antibody in the sample is bound to the PC and/or the PC-conjugate, and then a labeled detection reagent, typically an anti-immunoglobulin antibody, is bound to the antibody. The amount of labeled detection reagent bound to the antibody is then measured. Unlike the competitive method, the results of the noncompetitive method will be directly proportional to the concentration of the antibody. In a noncompetitive immunoassay or western blot, a labeled detection reagent, typically an anti-immunoglobulin antibody, may be used to detect antibody bound to the PC and/or the PC-conjugate. A suitable anti-immunoglobulin antibody will bind specifically to immunoglobulin of the species from which the sample is obtained. It may bind to all immunoglobulin isotypes of that species, or only a subset of isotypes. For example, it may bind only to IgA, IgD, IgE, IgG or IgM, or combinations of two or more of these isotypes. Biding to IgM may be preferred. The anti-immunoglobulin antibody may bind specifically only to certain subtypes of any given isotype. Subtypes of human IgA are IgA1 and IgA2. The anti-immunoglobulin antibody may bind to one or both of these subtypes. Subtypes of human IgG are IgG1, IgG2, IgG3 and IgG4. The anti-immunoglobulin may bind to one or more of these human IgG subtypes. Subtypes of human IgM are IgM1 and IgM2. The anti-immunoglobulin antibody may bind to one or both of these subtypes. It will be appreciated that there are different isotypes and subtypes in different vertebrate species.

In radioimmunoassay, the antibody or detection reagent is labeled with a radioisotope, such as ¹³¹I or ¹²⁵I. In enzyme immunoassays, the antibody or detection reagent is labeled with an enzyme. Suitable enzymes include those that are capable of being detected with the use of a chromogenic substrate. A chromogenic substrate is a substance which, as a result of the reaction with the enzyme, gives rise to a coloured product which can thus be detected spectrophotometrically. Enzymes such as horse radish peroxidase, alkaline phosphatase, beta-galactosidase, and pyrophosphatase from *E. coli* have been widely employed. Chemi-luminescent systems based on enzymes such as luciferase can also be used. Other labels include fluorescent labels such as fluorophores of the Alexa series.

Conjugation of the antibody or detection reagent with the vitamin biotin is frequently used since this can readily be detected by its reaction with enzyme- or fluorophore-linked avidin or streptavidin to which it binds with great specificity and affinity.

In a typical non-competitive enzyme immunoassay, the sample to be analyzed is placed in contact and incubated with the PC and/or the PC-conjugate adsorbed on a solid substrate. Any anti-PC antibodies that are possibly present in the sample are thus specifically bound by the PC and/or the PC-conjugate adsorbed on the solid substrate, producing a complex between the anti-PC and PC and/or the PC-conjugate. The sample is then separated from the solid substrate so as to eliminate non-bound materials, for example, by washing. In the next step of the method, an indicator antibody capable of binding any anti-PC antibodies that are present on the substrate in the form of the complex is added to the solid substrate, thus producing a complex between the anti-PC, the PC and/or the PC-conjugate, and the indicator. The indicator antibody may, for example, be an anti-human IgM or IgG immunoglobulin raised in a non-human animal species. Finally, the presence of the complex between the anti-PC, the PC and/or the PC-conjugate, and the indicator on the solid substrate is detected, the presence of said complex on the solid substrate being indicative of the presence of anti-PC antibodies in the sample from the individual.

Typically, the solid substrate is a micro-titration plate, for example, of the type commonly used for performing ELISA immunological assays. The micro-titration plate is preferably a polystyrene plate. Other suitable solid substrates are latex particles, beads and coated red blood cells. Conveniently, the PC and/or the PC-conjugate is adsorbed to the solid substrate by incubating the PC and/or the PC-conjugate in a buffer with the solid substrate. Suitable buffers include carbonate buffer or phosphate buffered saline. Alternatively, the PC and/or the PC-conjugate may be covalently linked to the solid substrate. Typically, after adsorption or covalent linkage of the PC and/or the PC-conjugate to the solid substrate, the solid substrate is incubated with a blocking agent to reduce non-specific binding of matter from the sample to the solid substrate. Suitable blocking agents include bovine serum albumin.

It is preferred that a quantitative estimate of antibody which can bind to the PC and/or the PC-conjugate is obtained by one or more of the above techniques. In typical non-competitive assays, a linear relationship between the measured variable, whether it be optical density or some other read-out, and antibody concentration, may be assumed. For example, if sample A has double the optical density of sample B in the assay (background having been subtracted from both), it may be assumed that the concentration of antibody is double in A compared to B. However, it is preferable to construct a standard curve of serial dilutions of a pool of positive serum samples. Preferably, such dilutions are assayed at the same time as the test samples. By doing this, any variation from the linear relationship may be taken into account in determining the quantity of antibody in the samples.

A particularly preferred immunoassay for assessing the levels of antibodies to PC and/or the PC-conjugate in a sample uses the commercially available CVDefine^{™} assay kit available from Athera Biotechnologies AB. THE CVDefine^{™} assay has been discussed in numerous publications, such as Grönlund et al, 2009; de Faire et al, 2008^{a}; Dahlbom et al, 2009; de Faire et al, 2008^{b}; Frostegård et al, 2007; Sjöberg et al, 2008^{a}; Sjöberg et al, 2008^{b}; and Su et al, 2006.

The CVDefine^{™} assay is an indirect non-competitive enzyme immunoassay for quantitative determination of anti-phosphorylcholine (anti-PC) IgM antibodies in human serum or plasma. The wells of a microplate are coated with PC antigen. PC-specific IgM antibodies present in the patient sample bind to the antigen. In a second step an enzyme labelled second antibody (conjugate) binds to the antigen-antibody complex which leads to the formation of an enzyme labelled conjugate-antibody-antigen complex. The enzyme labelled antigen-antibody complex converts the added substrate to form a coloured solution. The rate of colour formation from the chromogen is a function of the amount of conjugate complexed with the bound antibody and thus is proportional to the initial concentration of the respective antibodies in the patient sample. The CVDefine^{™} kit contains the following reagents -
- Microplate Strips: 12 strips x 8 wells (for 96 determinations) coated with PC conjugated to bovine serum albumin (BSA), maintained in a foil pouch containing desiccant.
- Calibrators: vials of anti-PC IgM antibody calibrators at concentrations of 0-6.25-12.5-25-50-100 U/ml in a buffer containing BSA, 0.095% (w/v) sodium azide, detergent and human serum, 1.5 ml each, ready to use.
- Control High: a vial of buffer containing BSA, 0.095% (w/v) sodium azide, detergent and human serum, 1.5 ml, ready to use. The level of IgM anti-PC in this control should be high enough to provide an optical density of greater than 0.6 when assayed by the following procedure.
- Control Low: a vial of buffer containing BSA, 0.095% (w/v) sodium azide, detergent and human serum, 1.5 ml, ready to use. The level of IgM anti-PC in this control should be low enough to provide an optical density of less than 0.2 when assayed by the following procedure.
- Wash Buffer Concentrate: a vial of 20x PBS concentrate and detergent, 75 ml
- Sample Diluent: a vial of PBS containing BSA, with <0.1% (w/v) sodium azide and detergent (Tween 20), 100 ml (yellow coloured), ready to use.
- IgM HRP Conjugate: a vial of anti-human IgM HRP (horse-radish peroxidase) from goat, 20 ml, ready to use.
- Substrate TMB: a vial of TMB (3, 3', 5, 5' Tetramethylbenzidine), < 0.05 % (w/v) in 20 ml water, ready to use,
- Stop Solution: a vial of 0.5 M H₂SO₄, 20 ml (colourless), ready to use

The CVDefine^{™} assay procedure is as follows -
(i) Dilute serum/plasma (1:101) using Sample Diluent.
(ii) Remove microplate strips from pouch and put firmly into strip holder. Plates are coated with PC-BSA (10 µg/mL) 50 µL/well in phosphate-buffered saline (PBS). Coated plates were incubated overnight at 4°C. After washing with PBS, the plates were blocked with 2% BSA/PBS for 2 hours at room temperature and washed with PBS.
(iii) Dispense 100 µl of calibrators, high and low controls and diluted patient samples into appropriate wells.
(iv) Incubate for 30 minutes.
(v) Aspirate fluid from wells and wash wells 3 times with Wash Buffer with soaking steps in between, as follows. Dispense 300 µl of 1 x Wash Buffer into each well and incubate for 20 seconds; remove Wash Buffer from the wells by aspiration or by inverting the plate over a sink and vigorously shaking ; remove residual Wash Buffer by tapping the inverted plate on clean absorbent paper. Repeat procedure 2 further times.
(vi) Dispense 100 µl of IgM HRP Conjugate into all wells.
(vii) Incubate for 30 minutes.
(viii) Aspirate fluid from wells and wash wells 3 times with Wash Buffer with soaking steps in between (see step (v) above).
(ix) Dispense 100 µl of Substrate TMB into all wells.
(x) Incubate for 10 minutes in the dark.
(xi) Dispense 50 µl of Stop Solution into all wells.
(xii) Read absorbance (OD) at 450 nm, max. 30 minutes after adding the Stop Solution. A reference wavelength of 620 nm is recommended.

The direct results of the CVDefine™ assay are initially expressed in arbitrary units. Calibrators and controls are adjusted and traceable to an in-house human reference serum preparation established at Athera Biotechnologies AB. The assay has a measuring range of 6.25 U/ml - 100 U/ml and a detection limit of 0.5 U/ml.

For quantitative evaluation of the results from this type of assay, an individual calibration must be performed for each run. Quantitative evaluation can be performed manually or by the use of data reduction software.

The manual procedure requires calculation of the mean absorbance (OD) value of each calibrator, which is plotted against the calibrator concentrations of 0, 6.25, 12.5, 25, 50, 100 U/ml on suitable graph paper. A smooth curve is then drawn considering all calibrator points, and the concentrations of the samples can then be read from the calibration curve.

When using data reduction software, a suitable computer program is selected that uses a 4 parameter or Cubic Spline curve fitting algorithm. In case the implemented curve fitting algorithm is not able to automatically handle standards with 0 U/ml, assign a minimal value to Calibrator 1 (0 U/ml), which is derived one log scale below the Calibrator 2 (e.g. 0.625 U/ml for Calibrator 1). Samples which give absorbances above that of the highest Calibrator are out of range of this assay and should be stated as > 100 U/ml. Such samples should be diluted as appropriate and reassayed.

Quantile cut-offs can be based on the anti-PC distribution in control groups. The associations between serum levels of anti-PC and risk of Alzheimer's disease can be determined by conditional logistic regression models with calculation of odds ratios (ORs) and 95% confidence intervals (CI). Test samples and controls may be age and gender matched for by design of the study. To test for differences in means/medians between cases and controls, the t-test may be used for normally distributed variables and the Wilcoxon Rank sum test for non-normally distributed variables. The Chi-Square test (and Fisher's exact for small samples) may be used to test for differences in proportions. The non-parametric Spearman Rank Correlation Coefficient may be used to test for correlations. Linear trend in proportions may be assessed through the Cochran-Armitage trend test. Linear trend in ORs over quantiles may be assessed by the Score-test (a Cochran-Armitage trend test) of quantile included as a continuous variable in SAS PROC LOGISTIC. A two-tailed p-value < 0.05 may be considered as significant. SAS may be used for the statistical analyses (release 9.2, SAS Institutet Inc. Cary, NC).

The inventors have surprisingly demonstrated that, typically, low levels of antibodies with reactivity to PC and/or a PC-conjugate are indicative of an increased risk of developing Alzheimer's disease, and/or is at risk of actually having already developed Alzheimer's disease. Conversely, high levels of antibodies with reactivity to the PC and/or a PC-conjugate are indicative of a reduced risk of developing Alzheimer's disease, and/or a reduced risk of actually having already developed Alzheimer's disease.

The level of antibodies with reactivity to PC and/or a PC-conjugate determined for any given individual may be categorised as high or low by reference to the range observed in the wider population or test cohort. It may be appropriate to assess anti-PC levels blood samples taken from individuals in a cohort *before* the onset of Alzheimer's disease (incident cases) compared to three unrelated age- and sex-matched controls at blood draw (+/- 1 year), and/or to assess anti-PC levels blood samples taken from individuals in a cohort *after* the onset of disease (prevalent cases) compared to three unrelated age- and sex-matched controls at blood draw (+/- 1 year). It may be possible to match the controls to more than one test case and so the effective number of controls may therefore be less than 3 x number of cases. The total number of test and controls individuals in a suitable cohort may be greater than 100, such as about 200, 300, 400, 500, 600, 700, 800, 900 or 1000. Where a test case shows a level of anti-PC antibodies below the mean average, or below a particular percentile value determined with reference to the wider population or cohort, it may be categorised as a low level. Suitably, a low level may correspond to a value below the 25^{th} percentile, or below the 20^{th}, 10^{th} or 5^{th} percentile. A high level may for example, correspond to a value of above the 5^{th}, 10^{th}, 20^{th}, or 25^{th} percentile, or above the mean average level.

In practice, the skilled person will appreciate that any percentile value cut-off point can be used to indicate a low level of anti-PC that is associated with increased risk of developing, or having, Alzheimer's disease, so long as, when conditional logistic regression analysis is performed on the anti-PC levels generated from a test cohort: -
- the calculated odds ratio for all individuals within that percentile group is greater than 1 (indicating that a person having an anti-PC level within the levels associated with that percentile is more likely to develop, or have, Alzheimer's disease than a person having an anti-PC level above that percentile); and
- the p-value calculated from the anti-PC values for individuals within that percentile group is less than 0.05 and the 95% odds ratio confidence interval for that group provides a range in which the lower limit is above 1 (wherein such p-values and CI values indicate that the odds ratio value ascribed to individuals with anti-PC levels falling within that percentile group is statistically significant).

In practice, therefore, the skilled person can readily determine by statistical analysis of the data from a cohort, the highest percentile value for which anti-PC levels indicate a statistically significant risk of developing, or having, Alzheimer's disease, and can also calculate associated (and incrementally higher) hazard ratios for individuals with anti-PC levels falling within lower percentile values

Additionally, or alternatively, the level of antibodies with reactivity to PC and/or a PC-conjugate determined for any given individual may be categorised as high or low by reference to the absolute level of anti-PC antibody within a sample taken from that individual, in view of the inventors' findings. Thus, when the level of anti-PC is determined quantitatively, for example by using the CVDefine^{™} kit assay, the inventors have found that mean levels of anti-PC IgM levels in a population are typically about 40-50 U/ml (corresponding to about 4-5 µg/ml), and values in a sample at or below this level may be considered as being low. Levels of anti-PC IgM of, or below, about 25-20 U/ml (which corresponds to about 2.5-3 µg/ml) are typically representative of values below the about the 25^{th} percentile, and values under about 17 U/ml (which corresponds to about 1.7 µg/ml) antibody are typically representative of values below about the 10^{th} percentile. Thus, anti-PC levels, such as IgM anti-PC levels, in a sample at or below about 50, 40, 30, 25, 20, 19, 18, 17, 16, 15, 14, 13, 12, 11, 10 or less U/ml (wherein 1 U/ml is equal to about 100 ng of antibody per ml) may be associated with an increased risk of developing Alzheimer's disease, and/or as a marker of actually having already developed Alzheimer's disease.

An example of a method to determine the absence or presence and/or level of IgM antibodies with reactivity to a PC-conjugate which is related to an increased or decreased risk of developing Alzheimer's diseases is described below. Other methods known in the art can also be used. Similar methods may be used to determine the absence or presence and/or level of IgG or IgA antibodies with reactivity to a PC-conjugate.

Where an individual is characterised as possessing low levels of antibodies with reactivity to PC and/or a PC-conjugate, this information will assist in the diagnosis, or prognosis of increased risk of development or progression, of Alzheimer's disease.

A clinician may take other factors into account in arriving at a diagnosis or prognosis. Thus, for example, the results of the methods of diagnosis according to the present invention may be collated with the results for other, art-known, tests for risk of Alzheimer's disease, such as test for memory loss (particularly with regard to the capacity to memorise recently learned information), altered behaviour (such as to the capacity for attentiveness, planning, flexibility, abstract thinking, semantic memory, and/or apathy), cognitive tests, including the mini-mental state examination (MMSE), brain scans (such as CT, MRI, SPECT or PET) and/or analysis of cerebrospinal fluid for amyloid protein and/or tau proteins.

For example, dementia may be ascertained through a two-step procedure which entails, first, a cognitive screening and, second, diagnostic assessment of each suspected case (Gatz et al, 2003, Behav Genet, 33(2) 95-105). Screening and diagnostic assessment have been described in detail previously (Dahl et al, 2007, Aging Clin Exp Res, 19(5):381-9), (Gatz et al, 1997, J Gerontol A Biol Sci Med Sc, 52(2), M117-25). In brief, the Mini-Mental State Examination (MMSE) (Folstein et al, 1975, J Psychiatr Res, 12, 189-198) can be used to screen for dementia. Those who screened positive for suspicion of dementia may be further evaluated through cognitive testing, clinical work-ups, informant interviews, reviews of medical records and laboratory tests. Final diagnoses of dementia may be set at a multidisciplinary consensus conference. Dementia may be diagnosed according to the criteria in the Diagnostic and Statistical Manual of Mental Disorders, 3rd edition, revised (DSM-III-R) (American Psychiatric Association 1987, ISBN 089042019X) and fourth edition (DSM-IV) (American Psychiatric Association 1994, ISBN 0890420246) and differentially diagnosed as Alzheimer's disease (based on the NINCDS/ADRDA criteria) (Dubois et al, 2007, Lancet Neurol, 6(8):734-46) vascular dementia (based on the NINDS-AIREN criteria) (Roman, 1993, Neurology, 43(2), 250-260), mixed dementia (AD with cerebrovascular disease), other specified dementia, or unspecified dementia.

Where the individual is determined to have, or have an increased risk of developing, Alzheimer's disease, then treatments (for example, treatments according to the present invention) and/or life-style changes (such as mental stimulation, exercise and/or dietary changes) may be recommended. Such treatments and/or life-style changes may be tailored to the individual.

### II. EXPERIMENTAL

The following examples are included to further illustrate various aspects of the invention. It should be appreciated by those of skill in the art that the techniques disclosed in the examples which follow represent techniques and/or compositions discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result.

### EXAMPLE 1

Study description. A case-control study of the association between serum levels of antibodies with reactivity to a PC-conjugate, anti-phosphorylcholine (anti-PC), IgM antibodies and the risk for dementia. PC is a component of the atherosclerotic plaque and low levels of anti-PC have in a previous study been associated with an increased risk of cardiovascular disease (Su et al., 2006 Atherosclerosis 188, 160-6). In the present study, the inventors have investigated the association between serum levels of anti-PC and the risk for 1) any dementia, 2) Alzheimer's disease (AD) and 3) vascular dementia (VaD).

The study is based on a sample of twins from the Swedish Twin Registry who participated in studies of aging and dementia. Dementia cases who gave blood ***before*** the onset of disease (***incident*** cases) and dementia cases who gave blood ***after*** the onset of disease (***prevalent*** cases) were matched to three unrelated controls on sex and age at blood draw (+/- 1 year). Controls could be matched to more than one case and the effective number of controls is therefore less than 3 x number of cases.

Study sample. The study sample consists of 842 twins. There are 179 incident dementia cases, 102 prevalent dementia cases and 561 controls. The sample also includes 61 complete twin pairs (of which 13 are monozygous [MZ]) discordant for (incident) dementia. To study the intra-individual variation of antiPC over time, the inventors also included a follow-up sample of serum on 23 randomly selected controls.

Analyses. Levels of anti-PC antibody was measured in serum with an ELISA procedure (CVDefine™, Athera Biotechnologies AB, as discussed above) with a detection limit of 0.5 Units per ml (U/ml). Test precision is established to less than 2.5 % (between assay variability) and less than 7 % (within assay variability) (from Athera CVDefine™ product sheet). Based on distribution of concentration of anti-PC antibody in the sample, twins were divided into quartiles (units of 25%). Odds ratio (OR) of dementia was modelled using conditional logistic regression with each strata including one case and matched controls.

Results - Descriptive statistics: *Case-confrol sample.* Mean age at blood draw for the full sample was 78.6 (range 52.6-94.3). Mean (geometric mean) of antiPC concentration was 48.2 U/ml, with quartiles <=28.5, 28. 5-46.6, 46.6-75.9, >75.9. Blood samples on incident cases were collected on average 4.6 (range 0.1-15.0) years before dementia onset. A description of the incident and prevalent case-control samples can be found in Tables 1 and 2.

**Table 1. Characteristics of incident dementia cases and matched controls.**

| | *Controls* | *Demented* | *AD*^{†} | *VaD* + *mixed*^{†} |
|---|---|---|---|---|
| | *n=377* | *n=179* | *n=91* | *n=56* |
| Age blood draw^{a} | 78.5 (52.6-93.0) | 78.9 (53.2-94.3) | 80.3 (60.1-94.3) | 76.8 (53.2-89.5) |
| Dementia onset^{a} | - | 83.5 (63-96) | 84.1 (63-96) | 82.0 (66-94) |
| Concentration | 50.6 | 51.6 | 46.7 | 62.7 |
| antiPC^{b} | (2.2) | (2.5) | (2.6) | (2.6) |
| antiPC- Quartiles^{c} | | | | |
| First (<=30.0) | 22.8 | 29.6 | 36.3 | 23.2 |
| Second (30.0-47.8) | 26.0 | 22.9 | 18.7 | 21.4 |
| Third (47.8-79.4) | 28.1 | 18.4 | 20.9 | 16.1 |
| Fourth (>79.4) | 23.1 | 29.1 | 24.2 | 39.3 |

^{a}Mean age in years, range in parenthesis, ^{b}Geometric mean concentration in U/ml, std in parenthesis, ^{c}Presented values are percentages belonging to each quartile. Quartiles are based on the distribution of the whole incident sample (N=556). ^{†}AD (Alzheimer's Disease), VaD (Vascular dementia) and mixed dementia are sub-categories to the Demented group.

**Table 2. Characteristics of prevalent dementia cases and matched controls.**

| | *Controls* | *Demented* | *AD^{†}* | *VaD* + *mixed^{†}* |
|---|---|---|---|---|
| | *n=205* | *n=102* | *n=74* | *n=28* |
| Age blood draw^{a} | 81.3 (67.0-93.0) | 81.7 (67.4-92.3) | 81.7 (69.2-92.3) | 81.8 (67.4-89.6) |
| Concentration | | | | |
| antiPC^{b} | 49.5 (2.2) | 39.2 (2.2) | 38.6(2.3) | 40.7(2.1) |
| | | | | |
| antiPC- Quartiles^{c} | | | | |
| First (<=26.8) | 21.0 | 33.3 | 36.5 | 25.0 |
| Second (26.8-43.6) | 24.9 | 25.5 | 20.3 | 39.3 |
| Third (43.6-69.3) | 27.3 | 20.6 | 21.6 | 17.9 |
| Fourth (>69.3) | 26.8 | 20.6 | 21.6 | 17.9 |

| | | | | |
|---|---|---|---|---|
| ^{a}Mean age in years, range in parenthesis, ^{b}Geometric mean concentration in U/ml, std in parenthesis, ^{c}presented values are percentages belonging to each quartile. Quartiles are based on the distribution of the whole prevalent case control sample (N=307). ^{†}AD (Alzheimer's Disease), VaD (Vascular dementia) and mixed dementia are subcategories to the Demented group. | | | | |

Results - Descriptive statistics: *Follow-up samples.* The follow-up sample was collected on average 3.1 years after the original sample (range 2.9-4.2). The concentration had decreased on average 6.6 U/ml (p=0.04), from 59.5 to 52.9 U/ml. There were 6 twins (30.4%) who changed from one antiPC concentration quartile to another from the first sample to the follow-up sample.

Results - Risk for dementia. The ORs of incident dementia, AD or VaD/mixed dementia can be seen in Table 3.

The risk of dementia and AD, when comparing those in the lowest quartile to the rest of the sample, was more prominent in those cases who gave blood 4 or more years before dementia onset, OR 1.86 (1.07-3.22), compared to those who gave blood less than four years before dementia onset, OR 1.29 (0.75-2.20). For AD, this effect appeared to be even more prominent with OR 4.83 (1.95- 11.96) and OR 1.20 (0.60-2.41) respectively. Table 4 shows the OR of prevalent dementia, AD or VaD/mixed dementia.

**Table 3. Odds ratios of incident dementia.**

| *antiPC (U ml)* | | *All dementias* | *AD* | *VaD* +*mixed* |
|---|---|---|---|---|
| Highest quartile vs rest | | 1.52 (1.04-2.23)* | 1.33 (0.76-2.34) | 2.01 (1.07-3.80)* |
| Lowest quartile vs rest | | 1.54 (1.05-2.26)* | 2.07 (1.22-3.51)* | 1.06 (0.53-2.12) |
| Categorical | Q2 | 0.61 (0.38-0.99)* | 0.40 (0.20-0.78)* | 0.75 (0.31-1.85) |
| | Q3 | 0.46 (0.28-0.76)* | 0.41 (0.21-0.80)* | 0.61 (0.24-1.57) |
| | Q4 | 0.99 (0.62-1.58) | 0.73 (0.38-1.43) | 1.55 (0.70-3.46) |

Quartiles are based on the cut-offs described in Table 1. For the analysis of anti- PC as a categorical variable, the lowest quartile (Q1) was used as the reference level. AD and VaD/mixed are sub-categories to All dementias. 95% CI in parenthesis. * indicates statistically significant results

**Table 4. Odds ratios of prevalent dementia.**

| *antiPC (U ml)* | | *All dementias* | *AD* | *VaD* + *mixed* |
|---|---|---|---|---|
| Highest quartile vs rest | | 0.74 (0.42-1.31) | 0.70 (0.36-1.37) | 0.87 (0.30-2.51) |
| Lowest quartile vs rest | | 1.85 (1.10-3.09)* | 2.78 (1.49-5.19)* | 0.70 (0.26-1.91) |
| Categorical | Q2 | 0.70 (0.38-1.29) | 0.45 (0.21-0.96)* | 1.80 (0.59-5.47) |
| | Q3 | 0.44 (0.23-0.84)* | 0.31 (0.15-0.66)* | 1.10 (0.28-4.29) |
| | Q4 | 0.47 (0.24-0.94)* | 0.33 (0.14-0.75)* | 1.09 (0.30-3.98) |

Quartiles are based on the cut-offs described in Table 2. For the analysis of antiPC as a categorical variable, the lowest quartile (Q1) was used as the reference level. AD and VaD/mixed are sub-categories to All dementias. 95% CI in parenthesis. * indicates statistically significant results.

Results - Descriptive statistics: *Co-twin control analyses* The OR of incident dementia based on the 48 dizygous (DZ) pairs was 1.09 (0.62-1.92). For the 13 MZ pairs the OR was 0.86 (0.29-2.55).

Discussion: There are no previous studies, to the best of our knowledge, on the link between any dementia, much less Alzheimer's disease, in humans or in animal models and anti-PC antibodies.

Natural anti-PC could be directly involved in the development of Alzheimer's disease through different albeit related mechanisms. We recently reported that anti-PC has anti-inflammatory properties, by inhibiting effects on endothelial activation induced by PAF and suggested that low levels of anti-PC could predispose to chronic inflammation and autoimmune disease, mediated by inflammatory, PAF-like and PC-exposing phospholipids (Su et al, 2008). It is therefore interesting to note that glycerophosphorylcholine is increased in AD brain (Barany et al, 1985; and Nitsch et al, 1992). and cerebrospinal fluid (Walter et al, 2004). These data imply that phosphatidylcholine hydrolysis could be raised in the brain in Alzheimer's disease, which in its turn is likely to be mediated by increased phospholipase activity, generating inflammatory phospholipids with exposed PC (Walter et al, 2004). Indeed, phospholipase A(2) (PLA(2)) enzyme may be involved in memory impairment and neurodegeneration in Alzheimer's disease (Schaeffer & Gattaz, 2008). Also oxidative stress is increased in Alzheimer's disease (Draczynska-Lusiak et al, 1998; Lovell & Markesbery 2007; and Pratico 2008), and low anti-PC could promote Alzheimer's disease by decreased inhibition of inflammatory phospholipids generated by PLA2 or oxidation.

Based on this study, we propose a novel paradigm, where the balance between proinflammatory factors like oxidized phospholipids and protective natural immunity as anti-PC is shifted, due to an immunodeficient state that could predispose to Alzheimer's disease. Raising anti-PC levels may be effective in delaying onset of or ameliorating symptoms of Alzheimer's disease.

All of the methods disclosed and claimed herein can be executed without undue experimentation in light of the present disclosure. While the methods of this invention have been described in terms of preferred embodiments, it will be apparent to those of skill in the art that variations may be applied to the methods, and in the steps or in the sequence of steps of the methods described herein.

### REFERENCES

U.S. Patent 5,455,032
WO 2005/100405
Barany et al, 1985, Lancet, 1(8427), 517
Binder et al., 2002. Nature Medicine 8, 1218-26.
Binder et al., 2003 Nature Medicine 9, 36-43.
Briles et al., 1982. J Exp Med 156, 1177-1185.
Brown et al., 1984. J Immunol 132, 1323-1328.
Caliguiri et al., 2007. J Am Coll Cardiol 50, 540-546.
Dahl A et al, 2007, Aging Clin Exp Res, 19(5):381-9
Dahlbom et al, 2009, Poster presentation at STAR
Dubois B et al, 2007, Lancet Neurol, 6(8):734-46
de Faire U et al, 2008a, Novel Inflammatory Cardiovascular Biomarkers - Clinical Implications Diabetes and Cardiovascular Risk, European Endocrinology Vol 4 Issue 2 Touch Briefings 2008
de Faire et al, 2008b, Poster presentation at ESC Sept 2, 2008
Draczynska-Lusiak et al, 1998, Mol Chem Neuropathol., 33(2), 139-148.
Faria-Neto et al., 2006. Atherosclerosis 189, 83-90.
Folstein MF et al, 1975, J Psychiatr Res, 72, 189-198
Frostegård et al, 2007, Nutr. Metab. (Lond)., 4(7), 1-10
Gatz M et al, 1997, J Gerontol A Biol Sci Med Sc, 52(2), M117-25
Gatz M et al, 2003, Behavior Genetics 33(2), 95-105
Grönlund et al, 2009, J. Cardiovascular Prevention and Rehabilitation, Published ahead of print, 14 April 2009, DOI 10.1097/HJR.0b013e32832a05df.
Kim et al., 2002 J Exp Med. 196, 655-65.
Lovell & Markesbery 2007, J Neurosci Res., 85(14), 3036-3040.
Nordenstam et al., 1990. Scand J Infect Dis 22,187-195.
Nitsch et al, 1992, Proc Natl Acad Sci U S A., 89(5), 1671-1675
Pratico 2008, Ann N Y Acad Sci., 1147, 70-78.
Roman GC et al, 1993, Neurology, 43(2), 250-260
Schenkein et al., 1999. Infect Immun 67, 4814-4818.
Schaeffer & Gattaz, 2008, Psychopharmacology (Berl)., 198(1),1-27
Shaw et al., 2000. J Clin Invest 105, 1731-1740.

## Claims

1. A method for assessing a human patient's risk of developing or progression of Alzheimer's disease, the method comprising assessing the patient's level of antibodies reactive with phosphorylcholine (PC) and/or a PC-conjugate in an *ex vivo* sample taken from the human patient, wherein the PC-conjugate comprises phosphorylcholine linked to a carrier, optionally via a spacer, and wherein the carrier is a protein, a carbohydrate, a lipid, a polymer, latex beads, or colloid metal.

2. The method of Claim 1 wherein low levels of the antibodies reactive with phosphorylcholine (PC) and/or a PC-conjugate are predictive of increased risk of developing or progression of Alzheimer's disease.

3. The method of any preceding claim wherein the method assesses the level of IgM, IgG and/or IgA antibodies reactive with phosphorylcholine (PC) and/or a PC-conjugate.

4. The method of any preceding claim wherein the level of antibodies reactive with phosphorylcholine (PC) and/or a PC-conjugate is assessed using a phosphorylcholine conjugate.

5. A method of diagnosing the presence or absence of IgM, IgG or IgA antibodies reactive with phosphorylcholine (PC) and/or a PC-conjugate related to increased or decreased risk of developing Alzheimer's disease in an *ex vivo* sample taken from a human patient, using a phosphorylcholine, conjugate, wherein the PC-conjugate comprises phosphorylcholine linked to a carrier, optionally via a spacer, and wherein the carrier is a protein, a carbohydrate, a lipid, a polymer, latex beads, or colloid metal.

6. The method of Claim 5 wherein low levels of the IgM, IgG or IgA antibodies reactive with phosphorylcholine (PC) and/or a PC-conjugate related to increased or decreased risk of developing Alzheimer's disease are predictive of increased risk of developing or progression of Alzheimer's disease.

7. The method of any preceding claim, wherein the PC-conjugate comprises phosphorylcholine linked to a carrier via a spacer.

8. The method according to any preceding claim wherein the PC-conjugate comprises phosphorylcholine linked to a carrier protein, and optionally wherein the protein is KLH (keyhole limpet hemocyanin), human serum albumin (HSA) or bovine serum albumin (BSA).

9. A use of a phosphorylcholine conjugate in an *ex vivo* method for assessing an individual's risk of developing or progression of Alzheimer's disease in which the individual's levels of antibodies, such as IgM, IgG or IgA antibodies, reactive with the phosphorylcholine conjugate are assessed, wherein the PC-conjugate comprises phosphorylcholine linked to a carrier, optionally via a spacer, and wherein the carrier is a protein, a carbohydrate, a lipid, a polymer, latex beads, or colloid metal.

## Patentansprüche

1. Ein Verfahren zur Einschätzung des Risikos von Entwicklung oder des Verlaufs von Alzheimerischer Krankheit eines menschlichen Patienten, das Verfahren umfassend Einschätzung des Pegels des Patienten von Antikörpern, die mit Phosphorylcholin (PC) und/oder einem PC-Konjugat in einer *ex vivo* Probe, entnommen von dem menschlichen Patienten, reagieren, wobei das PC-Kunjugat Phosphorylcholin, verbunden mit einem Träger, wahlweise über einen Abstandshalter, umfasst und wobei der Träger ein Protein, ein Kohlenhydrat, ein Lipid, ein Polymer, Latexperlen oder kolloides Metall ist.

2. Das Verfahren nach Anspruch 1, wobei niedrige Pegel von den Antikörpern, die mit Phosphorylcholin (PC) und/oder einem PC-Konjugat reagieren, vorhersagbar für erhöhtes Risiko von Entwicklung oder den Verlauf von Alzheimerischer Krankheit sind.

3. Das Verfahren nach einem vorhergehenden Anspruch, wobei das Verfahren den Pegel von IgM, IgG und/oder IgA Antikörpern, die mit Phosphorylcholin (PC) und/oder einem PC-Konjugat reagieren, einschätzt.

4. Das Verfahren nach einem vorhergehenden Anspruch, wobei der Pegel von Antikörpern, die mit Phosphorylcholin (PC) und/oder einem PC-Konjugat reagieren, unter Verwendung von Phosphorylcholin-Konjugat eingeschätzt wird.

5. Ein Verfahren zum Diagnostizieren der An- oder Abwesenheit von IgM, IgG oder IgA Antikörpern, die mit Phosphorylcholin (PC) und/oder einem PC-Konjugat reagieren, in Zusammenhang mit erhöhtem oder erniedrigtem Risiko der Entwicklung von Alzheimerischer Krankheit in einer *ex vivo* Probe, entnommen von einem menschlichen Patienten, unter Verwendung eines Phosphorylcholin-Konjugats, wobei das PC-Konjugat Phosphorylcholin, verbunden mit einem Träger, wahlweise über einen Abstandshalter, umfasst und wobei der Träger ein Protein, ein Kohlenhydrat, ein Lipid, ein Polymer, Latexperlen oder kolloides Metall ist.

6. Das Verfahren nach Anspruch 5, wobei niedrige Pegel von IgM, IgG oder IgA Antikörpern, die mit Phosphorylcholin (PC) und/oder einem PC-Konjugat reagieren, in Zusammenhang mit erhöhtem oder erniedrigtem Risiko von der Entwicklung von Alzheimerischer Krankheit, vorhersagbar für erhöhtes Risiko von Entwicklung oder den Verlauf von Alzheimerischer Krankheit sind.

7. Das Verfahren nach einem vorhergehenden Anspruch, wobei das PC-Konjugat Phosphorylcholin, verbunden mit einem Träger über einen Abstandshalter, umfasst.

8. Das Verfahren nach einem vorhergehenden Anspruch, wobei das PC-Konjugat Phosphorylcholin, verbunden mit einem Trägerprotein, umfasst, und wobei wahlweise das Protein KLH (Schlitzschnecken-Hämocyanin), menschliches Serumalbumin (HSA) oder Rinderserumalbumin (BSA) ist.

9. Eine Verwendung von einem Phosphorylcholin-Konjugat in einem *ex vivo* Verfahren zur Einschätzung des Risikos von Entwicklung oder des Verlaufs Alzheimerischer Krankheit eines Individuums, bei dem der Pegel des Individuums von Antikörpern, wie IgM, IgG oder IgA Antikörper, die mit dem Phosphorylcholin-Konjugat reagieren, wobei das PC-Konjugat Phosphorylcholin umfasst, verbunden mit einem Träger, wahlweise über einen Abstandshalter, eingeschätzt wird und wobei der Träger ein Protein, ein Kohlenhydrat, ein Lipid, ein Polymer, Latexperlen oder kolloides Metall ist.

## Revendications

1. Méthode d'évaluation du risque de développement ou de progression de la maladie d'Alzheimer chez un patient humain, la méthode comprenant l'évaluation du niveau d'anticorps réactifs avec la phosphorylcholine (PC) et/ou avec une conjugué-PC du patient dans un échantillon *ex vivo* provenant du patient humain, dans laquelle le conjugué-PC comprend une phosphorylcholine liée à un support, éventuellement par l'intermédiaire d'un espaceur, et dans laquelle le support est une protéine, un glucide, un lipide, un polymère, des billes de latex, ou un métal colloïdal.

2. Méthode selon la revendication 1 dans laquelle de bas niveaux d'anticorps réactifs avec la phosphorylcholine (PC) et/ou à un conjugué-PC sont prédicteurs d'un risque accru de développement ou de progression de la maladie d'Alzheimer.

3. Méthode selon l'une quelconque des revendications précédentes dans laquelle la méthode évalue le niveau d'anticorps IgM, IgG et/ou IgA réactifs avec la phosphorylcholine (PC) et/ou à un conjugué-PC.

4. Méthode selon l'une quelconque des revendications précédentes dans laquelle le niveau d'anticorps réactifs avec la phosphorylcholine (PC) et/ou à un conjugué-PC est évalué à l'aide d'un conjugué de phosphorylcholine.

5. Méthode destinée à diagnostiquer la présence ou l'absence d'anticorps IgM, IgG ou IgA réactifs avec la phosphorylcholine (PC) et/ou à un conjugué-PC liés au risque accru ou réduit de développement de la maladie d'Alzheimer dans un échantillon *ex vivo* provenant d'un patient humain, à l'aide d'un conjugué de phosphoryl-choline, dans laquelle le conjugué-PC comprend une phosphorylcholine liée à un support, éventuellement par l'intermédiaire d'un espaceur, et dans laquelle le support est une protéine, un glucide, un lipide, un polymère, des billes de latex, ou un métal colloïdal.

6. Méthode selon la revendication 5 dans laquelle de bas niveaux d'anticorps IgM, IgG ou IgA réactifs avec la phosphorylcholine (PC) et/ou à un conjugué-PC liés au risque accru ou réduit de développement de la maladie d'Alzheimer sont prédicteurs d'un risque accru de développement ou de progression de la maladie d'Alzheimer.

7. Méthode selon l'une quelconque des revendications précédentes, dans laquelle le conjugué-PC comprend une phosphorylcholine liée à un support par l'intermédiaire d'un espaceur.

8. Méthode selon l'une quelconque des revendications précédentes, dans laquelle le conjugué-PC comprend une phosphorylcholine liée à une protéine porteuse, et éventuellement dans laquelle la protéine est une KLH (hémocyanine de la fissurelle), une sérumalbumine humaine (HSA) ou une sérumalbumine bovine (BSA).

9. Utilisation d'un conjugué de phosphorylcholine dans une méthode ex *vivo* de progression du risque de développement ou d'évolution de la maladie d'Alzheimer chez un patient humain, dans laquelle les niveaux d'anticorps du patient, tels que les anticorps IgM, IgG ou IgA, réactifs avec le conjugué de phosphoryl-choline sont évalués, dans laquelle le conjugué-PC comprend une phosphorylcholine liée à un support, éventuellement par l'intermédiaire d'un espaceur, et dans laquelle le support est une protéine, un glucide, un lipide, un polymère, des billes de latex, ou un métal colloïdal.
